(19) Europäisches Patentamt
European Patent Office
Office européen des brevets

(11) Publication number: **0 079 772**
**A2**

(12) # EUROPEAN PATENT APPLICATION

(21) Application number: 82306030.6

(22) Date of filing: 12.11.82

(51) Int. Cl.³: C 07 D 251/16
C 07 D 251/22, C 07 D 251/44
C 07 D 251/46, C 07 D 251/50
C 07 D 251/52, C 07 D 239/42
C 07 D 239/46, C 07 D 239/48
C 07 D 239/52, C 07 D 491/04
//(C07D491/04, 311/00, 239/00),
(C07D491/04, 307/00, 239/00)

(30) Priority: 13.11.81 US 321284
13.11.81 US 321285

(43) Date of publication of application:
25.05.83 Bulletin 83/21

(84) Designated Contracting States:
DE FR GB IT

(71) Applicant: PPG INDUSTRIES, INC.
One Gateway Center
Pittsburgh Pennsylvania 15222(US)

(72) Inventor: Richter, Sidney Bernard
2601 Sand Run Parkway
Fairlawn Ohio 44313(US)

(72) Inventor: Krass, Dennis Keith
507 Dan Avenue
Canal Fulton Ohio 44614(US)

(74) Representative: Shipton, Gordon Owen et al,
W.P. THOMPSON & CO. Coopers Building Church Street
Liverpool L1 3AB(GB)

(54) **Herbicidally active 1-(2-oximo-phenyl)-3-heterocyclyl urea derivatives.**

(57) Compounds represented by the formula:

I.

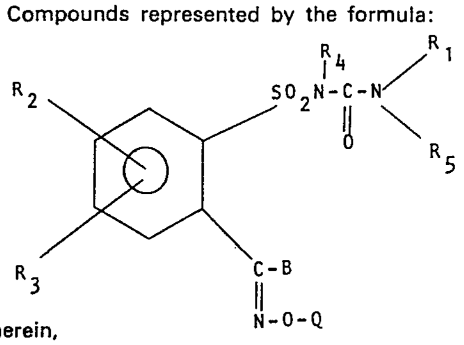

wherein,
$R^1$ is a group structurally represented by the Formulae II through VII:

II

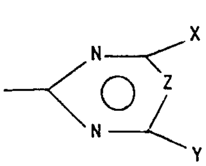

;

III

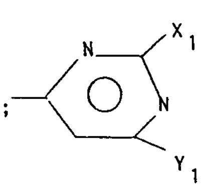

;

IV

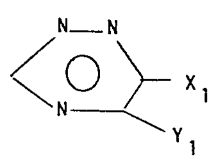

;

V

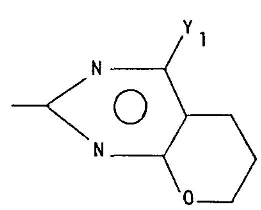

VI

;

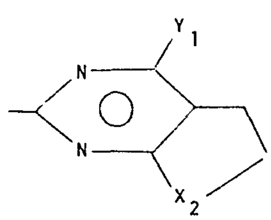

;

VII

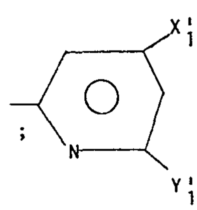

EP 0 079 772 A2

DESCRIPTION

"HERBICIDALLY ACTIVE 1-(2-OXIMO-PHENYL)-3-HETEROCYCLYL UREA DERIVATIVES"

This invention relates to certain 1-(2-oximo-phenyl)-3-heterocyclyl urea derivatives structurally represented by the Formula I:

I. 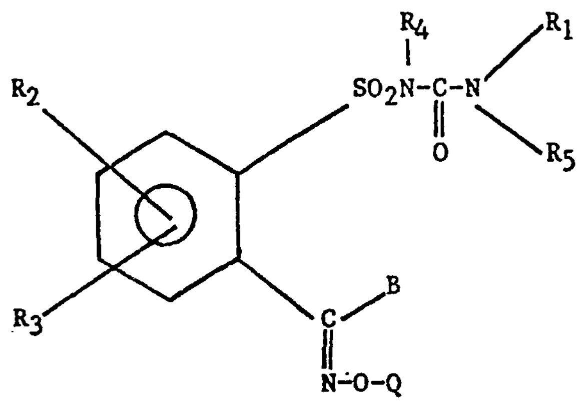

wherein,

$R^1$ is a group structurally represented by the Formulae II through VII:

II

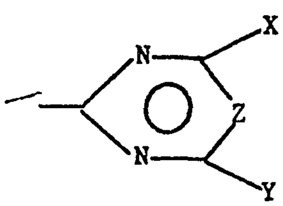

;

III

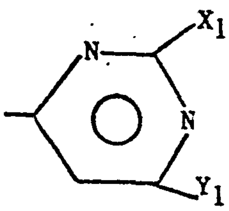

;

IV

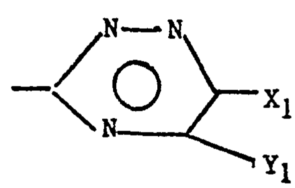

;

V

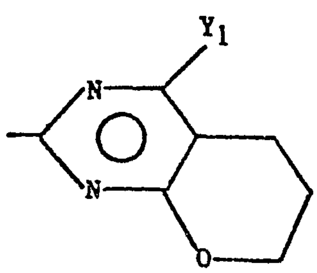

;

VI

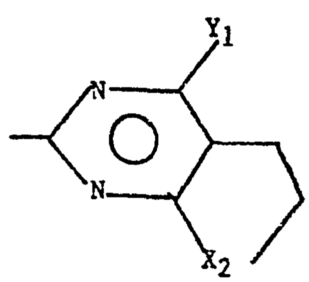

;

VII

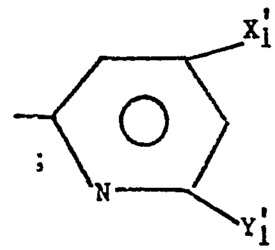

wherein

X is H, Cl, Me, MeO, EtO or $MeO(CH_2)_2O$;

Z is CH, N, CF, CCl, CBr, CMe, COMe, CEt or $C-(CH_2)_2Cl$;

Y is H, Cl, $C_1$ to $C_4$ alkyl which may be substituted by MeO, EtO, CN, $CO_2Me$, $CO_2Et$, or 1 to 3 F, Cl or Br; $C_3$ to $C_4$ alkenyl; $CR_6{\equiv}(CH_2-$; $-A(CH_2)_m-A_1-(C_1$ to $C_3)$ alkyl; $-A-CH_2-COL$; $-A-CHMe-COL$; $-A(CH_2-COL$; -SCN; $N_3$, $NR_7R_8$, $OR_9$ or $SR_{10}$;

$R_6$ is H, Me or $CH_2Cl$;

m is 2 or 3;

A is O or S;

$A_1$ is O, S or $SO_2$;

L is OH, $NH_2$, -NMe-OMe, mono- or di-($C_1$ to $C_4$ alkyl) amino or $C_1$ to $C_6$ alkoxy;

$R_7$ is H or Me;

$R_8$ is H, MeO, $C_1$ to $C_4$ alkyl, $C_3$ to $C_6$ cycloalkyl, $C_1$ to $C_4$ alkyl substituted by CN, $CO_2Me$ or $CO_2Et$, $C_3$ to $C_4$ alkenyl, or $C_2$ to $C_3$ alkyl substituted by MeO or EtO; or $R_7$ and $R_8$ combine to form $(CH_2)_4$ or $(CH_2)_2-O-(CH_2)_2$;

$R_9$ is $C_1$ to $C_4$ alkyl; $C_2$ to $C_4$ alkyl substituted by 1 to 3 F, Cl or Br; $C_1$ to $C_4$ alkyl substituted by CN, $C_3$ to $C_4$ alkenyl, $CR_6{\equiv}C-CH_2$-cyclopropylmethyl or 3-tetrahydrofuryl;

$R_{10}$ is $C_1$ to $C_4$ alkyl, or $C_1$ to $C_2$ alkyl substituted by CN, allyl or propargyl;

$X_1$ is H, Cl, MeO, EtO or Me;

$Y_1$ is H, MeO or Me;

$X_2$ is O or $CH_2$;

$X_1'$ and $Y_1'$ are Me or MeO;

$R_2$ is H, F, Cl, Br, $C_1$ to $C_4$ alkyl, $NO_2$, $CH_3SO_2-$, MeO, MeS, $CF_3$, $(CH_3)_2N-$, $NH_2$ or CN;

$R_3$ is H, F, Cl, Br or $CH_3$;

$R_4$ is H or $CH_3$;

$R_5$ is H, $CH_3$ or $OCH_3$;

B is H, or $C_1$ to $C_4$ alkyl, or cyano;
Q is $CH_2CO_2W$ or $CH(CH_3)CO_2W$; and
W is H, $C_1$ to $C_{10}$ alkyl or a cation.

Compounds of this invention embodied in the Formula I are very active broad spectrum herbicides and are effective in regulating growth of a wide variety of undesirable plants, i.e., weeds, when applied, in herbicidally effective amount, to the growth medium prior to emergence of the weeds or to the weeds subsequent to emergence from the growth medium. The term "herbicidally effective amount" is that amount of compound or mixture of compounds of this invention required to so injure or damage weeds such that the weeds are incapable of recovering following application. The quantity of compound or mixture of compounds of this invention applied in order to exhibit a satisfactory herbicidal effect may vary over a wide range and depends on a variety of factors, such as, for example, hardiness of a particular weed specifies, extent of weed infestation, climatic conditions, soil conditions, method of application and the like. Typically, as little as 0.1 or less pound per acre of compound or mixture of compounds of this invention would be expected to provide satisfactory weed control, although in some instances application rates in excess of one pound per acre might be required. Of course, the efficacy of a particular compound against a particular weed species may readily be determined by routine laboratory or field testing in a manner well known to the art.

A compound or compounds of this invention may, of course, be used as such or in formulation with agronomically acceptable adjuvants, inert carriers, other herbicides, or other commonly used agricultural compounds, for example, insecticides, fungicides,

stabilizers, safeners, fertilizers or the like.
The compounds of this invention alone or in formulation with other agronomically used materials are typically applied in the form of dusts, granules, wettable powders, solutions, suspensions, aerosols,emulsions, dispersions or the like in a manner well known to the art. When formulated with other typically used agronomically acceptable materials, the amount of compound or compounds of this invention may vary over a wide range, for example, from about 0.05 to about 95 percent by weight on weight of formulation. Typically, such formulations would contain from about 5 to about 75 percent by weight of compound or compounds of this invention.

A compound or compounds of this invention are effective in controlling a variety of common broadleaved and grassy weeds at application rates of only a few grams per acre either pre- or post-emergent. Exemplary of weeds that may be effectively controlled by the application of compounds of this invention are barnyard grass (Echinochloa crusgalli), crabgrass (Digitaria sauguinalis), coffeeweed (Daubentonia punices), jimsonweed (Datura stamonium), johnsongrass (Sorghum halepense), tall morning glory (Ipomoea purpurea), wild mustard (Brassica caber), teaweed (Sida Spinosa), velvetleaf (Abutilon Theophrasti), wild oats (Avena fatua), yellow foxtail (Setaria glauca), yellow nutsedge (Cyperus esculentus) and the like.

Compounds of this invention may be prepared for example by reacting an oxime compound of the Formula VIII:

VIII.

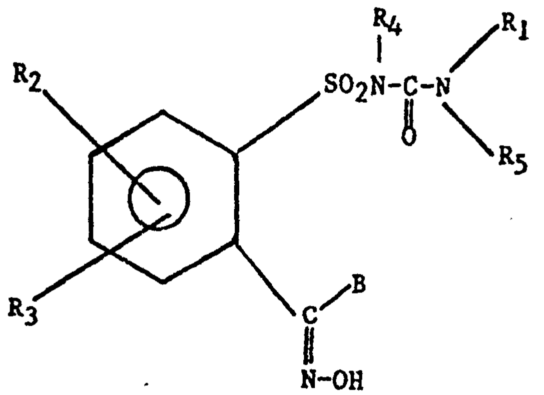

wherein $R_1$, $R_2$, $R_3$, $R_4$, $R_5$ and B are as defined with reference to the compounds of Formula I, with at least an equimolar amount of an α-halocarboxylate of the formula, Hal-Q, wherein Hal is halogen, preferably bromine or chlorine and Q is as defined with reference to the compounds of Formula I. The reaction between the Formula VIII compound and the α-halocarboxylate is typically conducted in the presence of an inert solvent at a temperature ranging from ambient to reflux and the reaction mixture worked-up in known fashion to isolate the compound of this invention.

Alternatively, compounds of this invention may be prepared for example by reacting an aldehyde or ketone compound of the formula IX:

IX.

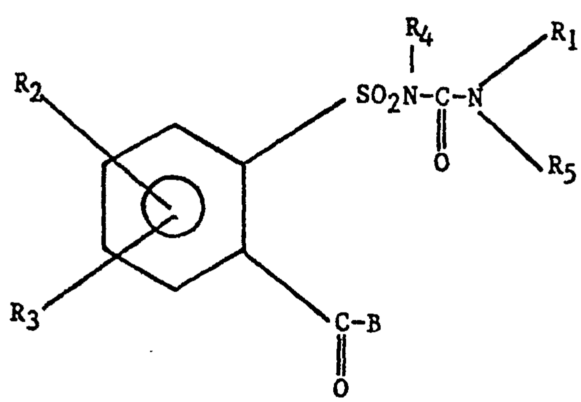

wherein $R_1$, $R_2$, $R_3$, $R_4$, $R_5$ and B are as defined with reference to the compounds of Formula I, with at least an equimolar amount of an aminoxycarboxylate of the formula $NH_2O-Q$, wherein Q is as defined with reference to the compounds of Formula I. The reaction between the compound of Formula IX and the aminoxycarboxylate is typically conducted in the presence of an inert solvent at a temperature ranging from ambient to reflux and the reaction mixture worked-up in known fashion to isolate a compound of the invention.

Regarding starting materials for the above described preparations, the $\alpha$-halocarboxylate and aminoxycarboxylate can be obtained from commercial sources or synthesized by known techniques, whereas the preparation of compounds of the Formulae VIII and IX is described in European Patent Application Publication No. 0030139 the teachings of which are incorporated by reference herein as regards the mode of preparation of said compounds.

Although the invention has been described in some detail with reference to certain embodiments thereof, it is to be understood that it is not intended to be so limited, since many variations may be made therein by those skilled in the art without departing from the spirit and scope thereof, except as defined in the appended claims.

## CLAIMS

1. A compound represented by the formula:

I. 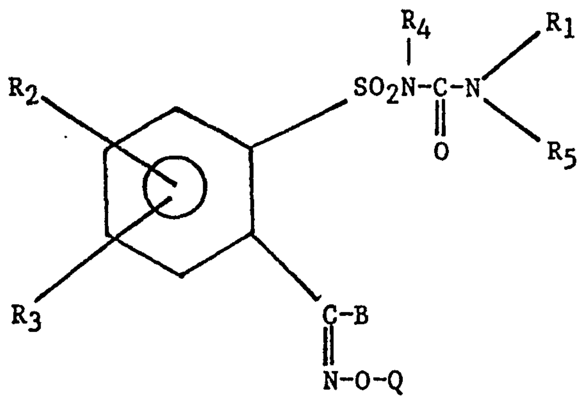

wherein,

$R^1$ is a group structurally represented by the Formulae II through VII:

II
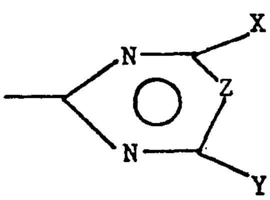
;
III
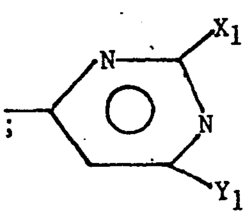
;
IV
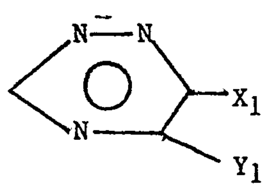
;

V
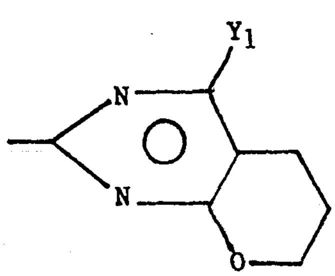
;
VI
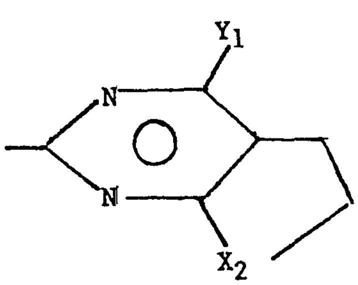
;
VII
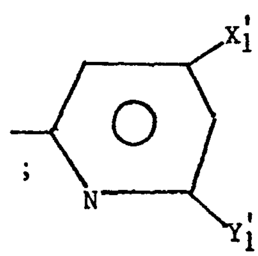

wherein

X is H, Cl, Me, MeO, EtO or $MeO(CH_2)_2O$;

Z is CH, N, CF, CCl, CBr, CMe, COMe, CEt or $C-(CH_2)_2Cl$;

Y is H, Cl, $C_1$ to $C_4$ alkyl which may be substituted by MeO, EtO, CN, $CO_2Me$, $CO_2Et$, or 1 to 3 F, Cl or Br; $C_3$ to $C_4$ alkenyl; $CR_6{\equiv}(CH_2-$; $-A(CH_2)_m-A_1-(C_1$ to $C_3)$ alkyl; $-A-CH_2-COL$; $-A-CHMe-COL$; $-A(CH_2-COL$; $-SCN$; $N_3$, $NR_7R_8$, $OR_9$ or $SR_{10}$;

$R_6$ is H, Me or $CH_2Cl$;

m is 2 or 3;

A is O or S;

$A_1$ is O, S or $SO_2$;

L is OH, $NH_2$, -NMe-OMe, mono- or di-($C_1$ to $C_4$ alkyl) amino or $C_1$ to $C_6$ alkoxy;

$R_7$ is H or Me;

$R_8$ is H, MeO, $C_1$ to $C_4$ alkyl, $C_3$ to $C_6$ cycloalkyl, $C_1$ to $C_4$ alkyl substituted by CN, $CO_2Me$ or $CO_2Et$, $C_3$ to $C_4$ alkenyl, or $C_2$ to $C_3$ alkyl substituted by MeO or EtO; or $R_7$ and $R_8$ combine to form $(CH_2)_4$ or $(CH_2)_2-O-(CH_2)_2$;

$R_9$ is $C_1$ to $C_4$ alkyl; $C_2$ to $C_4$ alkyl substituted by 1 to 3 F, Cl or Br; $C_1$ to $C_4$ alkyl substituted by CN, $C_3$ to $C_4$ alkenyl, $CR_6{\equiv}C-CH_2$-cyclopropylmethyl or 3-tetrahydrofuryl;

$R_{10}$ is $C_1$ to $C_4$ alkyl, or $C_1$ to $C_2$ alkyl substituted by CN, allyl or propargyl;

$X_1$ is H, Cl, MeO, EtO or Me;

$Y_1$ is H, MeO or Me;

$X_2$ is O or $CH_2$;

$X_1'$ and $Y_1'$ are Me or MeO;

$R_2$ is H, F, Cl, Br, $C_1$ to $C_4$ alkyl, $NO_2$, $CH_3SO_2-$, MeO, MeS, $CF_3$, $(CH_3)_2N-$, $NH_2$ or CN;

$R_3$ is H, F, Cl, Br or $CH_3$;

$R_4$ is H or $CH_3$;

$R_5$ is H, $CH_3$ or $OCH_3$;

B is H, or $C_1$ to $C_4$ alkyl, or cyano;
Q is $CH_2CO_2W$ or $CH(CH_3)CO_2W$; and
W is H, $C_1$ to $C_{10}$ alkyl or a cation.

2. A compound according to claim 1 wherein Q is $CH_2CO_2W$ or $CH(CH_3)CO_2W$ wherein W is $C_1$ to $C_{10}$ alkyl.

3. A compound according to claim 2 wherein W is methyl.